(19) Europäisches Patentamt
European Patent Office
Office européen des brevets

(11) Veröffentlichungsnummer : **0 347 621 B1**

# (12) EUROPÄISCHE PATENTSCHRIFT

(45) Veröffentlichungstag der Patentschrift : **06.05.92 Patentblatt 92/19**

(51) Int. Cl.$^5$ : **C07C 53/124,** C07C 51/42, C07C 69/24, C07C 67/48

(21) Anmeldenummer : **89109798.2**

(22) Anmeldetag : **31.05.89**

## (54) Verfahren zur Abtrennung von Fluorwasserstoff aus Isobuttersäure oder ihren Alkylestern.

(30) Priorität : **23.06.88 DE 3821155**

(43) Veröffentlichungstag der Anmeldung : **27.12.89 Patentblatt 89/52**

(45) Bekanntmachung des Hinweises auf die Patenterteilung : **06.05.92 Patentblatt 92/19**

(84) Benannte Vertragsstaaten : **AT BE CH DE ES FR GB IT LI NL SE**

(56) Entgegenhaltungen :
**EP-A- 0 031 886**
**EP-A- 0 079 497**
**EP-A- 0 091 604**
**US-A- 4 661 296**

(73) Patentinhaber : **RÖHM GMBH**
**Kirschenallee**
**W-6100 Darmstadt (DE)**

(72) Erfinder : **Ruppert, Wolfgang, Dr.Ing.**
**Am Mühlgraben 9**
**W-6101 Bickenbach (DE)**

EP 0 347 621 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft die Gewinnung von Isobuttersäure oder ihren Alkylestern nach deren Synthesen unter Verwendung von Fluorwasserstoff als Kochschem Katalysator und insbesondere die Abtrennung von bei der Trennung Fluorwasserstoff-Isobuttersäure bzw. ihrer Ester durch Destillation nicht sauber abgetrenntem Fluorwasserstoff aus der organischen Säure bzw. den Estern.

Stand der Technik

Die Umsetzung von Propylen, Kohlenmonoxid und Wasser nach der Kochschen Synthese in Gegenwart von Fluorwasserstoff als saurem Katalysator zu Isobuttersäure wurde von Y. Takezaki ("Bulletin of The Japan Petroleum Institute", Vol.8, 1966, 31 bis 38) eingehend untersucht.

Ausgestaltungen dieser Synthese für eine technische Herstellung von Isobuttersäure oder ihrer Ester sind beschrieben in dem EP 0 031 886, das eine kontinuierliche Arbeitsweise und Rückführung von wiederverwertbaren Nebenprodukten angibt, und in dem EP 0 091 604, das die kontinuierliche Herstellung von Isobuttersäurefluorid und dessen Hydrolyse zu Isobuttersäure beinhaltet. Die Gewinnung von reiner Isobuttersäure wird, wie in beiden europäischen Patenten angegeben, durch Destillation erreicht.

Eine wesentliche Verwendung von Isobuttersäure oder ihrer Ester dürfte in ihrer Bedeutung als Ausgangsprodukte für eine technische Herstellung von Methacrylsäure und ihrer Ester, wie dem technisch so bedeutsamen Methylmethacrylat, liegen und die, wie auch in dem EP 0 031 886 beschrieben, durch oxidative Dehydrierung der Isobuttersäure(verbindungen) in Dampfform in einem Reaktor erhalten werden. Da diese katalytisch durchgeführte Reaktion und auch entsprechende Anlagenteile von Fluorwasserstoff gestört bzw. korrodiert werden können, ist es wichtig, den Fluorwasserstoff praktisch vollständig aus den Reaktionsprodukten der Kochschen Synthese zu entfernen. Zur restlosen Entfernung von Fluorwasserstoff schickt man nach den Angaben in EP 0 031 886 die dampfförmige Isobuttersäure über eine geeignete Absorptionssäule, beispielsweise mit Bauxitfüllung.

Die bei der Aufarbeitung anfallenden hochsiedenden oder nichtflüchtigen Destillationsrückstände können verbrannt werden, wonach der dabei entstehende Abgasstrom zur Abtrennung von gegebenfalls noch vorhandenem Fluorwasserstoff wie in dem EP 0 079 497 angegeben, durch eine nachgeschaltete Absorptionssäule mit einem basischen Absorptionsmittel, wie z.B. Bauxit, Kalk oder Kalkmilch, geleitet wird.
Beim Überleiten bzw. Durchleiten von Fluorwasserstoffhaltiger Isobuttersäure in diese basischen Absorptionsmittel enthaltenden Vorrichtungen, werden auch die isobuttersauren Salze der den Absorptionsmitteln zugrunde liegenden Basen, d.h. Calcium und Aluminiumsalze der Isobuttersäure, gebildet. Dadurch wird die Basenkapazität, die eigentlich zur Bindung des Fluorwasserstoffs benötigt wird, schnell erschöpft. Die Bildung großer Mengen von Ca-bzw. Al-isobutyrat macht so die HF-Entfernung ineffektiv und führt zu relativ großen Isobuttersäureverlusten. Eine Rückgewinnung der Isobuttersäure aus den Salzen wäre zwar möglich, doch ist dies sehr aufwendig und damit teuer.

Aufgabe und Lösung

Für die Abtrennung von Fluorwasserstoffresten aus Isobuttersäure oder ihren Alkylestern, wie sie bei der Synthese in Gegenwart von HF als Katalysator anfallen, und die durch Destillation aus dem Synthesegemisch gewonnen werden, sollte gegenüber dem geschilderten Stand der Technik ein verbessertes Verfahren gefunden werden. Eine der Destillation nachgeschaltete Feinabtrennung ist beispielsweise bei Betriebsstörungen der Rektifikation notwendig.

Es wurde gefunden, daß durch Behandeln von Isobuttersäure und auch von Isobuttersäureestern, die noch geringe und für Folgereaktionen noch störende Menge an HF enthalten, mit organischen Anionenaustauschern, HF an diesen gebunden wird, ohne daß, wie für den wichtigen Fall der HF-Isobuttersäure-Trennung, Isobuttersäure an dem Harz ionisch in merklichen Mengen gebunden wird. Das erfindungsgemäße Verfahren ist sinnvoll zur Abtrennung von HF aus Isobuttersäureestern und insbesondere aus Isobuttersäure bei HF-Gehalten bis in den Konzentrationsbereich von 0,5 bis 1 Gew.-% $F^{\ominus}$-Gehalt. Dabei wird $F^{\ominus}$-Gehalt auf < 5 ppm, d.h. bis auf einige ppm $F^{\ominus}$-Ionen, erniedrigt.

Die Erfindung betrifft daher ein:
Verfahren zur Abrennung von in Isobuttersäure oder ihren Estern in Mengen von etwa 5 bis 10 ppm bis etwa 1 Gew.-% enthaltendem Fluorwasserstoff durch Behandeln des Gemisches mit einer Base, dadurch gekennzeichnet, daß die Abtrennung mit einem organischen Anionenaustauscherharz als Base durchgeführt wird.

Durchführung der Erfindung

Das durch Rektifikation erhaltene Isobuttersäure- bzw. Isobuttersäureester-Kondensat mit einer Temperatur von etwa 10 bis 40 Grad C, in welchem eine kontinuierlich durchgeführte Messung, z.B. durch eine $F^{\ominus}$-sensitive Elektrode, einen für die weitere Verwendung untragbar hohen $F^{\ominus}$-Gehalt anzeigt, wird zur weitgehendsten Entfernng von HF mit einem basischen Ionenaustauscherharz behandelt.
Die basischen Ionenaustauscherharze besitzen wie bekannt als funktionelle Gruppen quarternäre Ammoniumgruppen - stark basische Anionenaustauscher - oder primäre, sekundäre oder tertiäre Aminogruppen - schwach basische Anionenaustauscher - und sind durch Polykondensations- und Polymerisationsverfahren herstellbar (Ullmanns Enzyklopädie der technischen Chemie, Band 13, 4. Auflage, Seiten 297 bis 312). Das erfindungsgemäße Verfahren wird vor allem mit schwach basischen Anionenaustauscherharzen durchgeführt. Beispielhaft seien Harze genannt, die aus Polystyrol als Grundgerüst bestehen und als funktionelle Gruppen, die auch als Ankergruppen bezeichnet werden, tertiäre Aminogruppen, wie

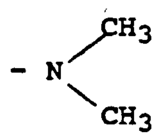 -Gruppen,

enthalten.
Ein Vertreter dieser erfindungsgemäß brauchbaren Anionenaustauscherharze ist das Handelsprodukt Lewatit® MP 62 von Bayer.
Vorteilhaft wird das Austauscherharz als Festbett in einer Säule bzw. technisch als Kolonne angewendet, auf welches z.B. die fluorwasserstoffhaltige Isobuttersäure am Kolonneneingang aufgegeben wird. Bei genügender Austauscherkapazität des Harzes wird am Ausgang der Kolonne ein Produkt mit einem Gehalt unter 5 ppm, insbesondere von 1 bis 3 ppm HF, erhalten. Die Temperatur im Austauscherharz kann bei dem erfindungsgemäßen Verfahren etwa zwischen 0 Grad C und 90 Grad C, insbesondere zwischen 5 Grad C und 60 Grad C, vor allem zwischen 10 Grad C und 40 Grad C liegen.
Sobald der Austauscher seiner Kapazität entsprechend beladen ist, wird in einem Regenerierungsprozeß, mit Durchleiten einer Base nach bekannten Verfahrensweisen, die Ausgangskapazität des Austauschers wiederhergestellt.

Das erfindungsgemäße Verfahren gestattet HF-Isobuttersäure-Gemische ohne Bildung schwierig zerlegbarer isobuttersaurer Salze zu trennen.

BEISPIEL

Durch 15 kg Anionenaustauscherharz Lewatit® MP 62, geschüttet in eine Säule von 2 m Höhe und einem Gesamtvolumen von 25 l, werden innerhalb von 50 Stunden 400 kg mit 1 300 bis 1 500 ppm $F^{\ominus}$ beladene Isobuttersäure von 25 Grad C geleitet. Die danach erhaltene Isobuttersäure hatte einen $F^{\ominus}$-Gehalt von < 5 ppm. Das verwendete Anionenaustauscherharz war nach der beschriebenen Ausführung noch nicht mit $F^{\ominus}$ vollständig belegt.

Die Regenerierung erfolgt wie in der Firmenschrift der Bayer AG "Orientierungshilfe zur optimalen Anwendung von Lewatit/Lewasorb unter Berücksichtigung der Verfahrensweise", Ausgabe Januar 1986, angegeben, mit verdünnter NaOH.

## Patentansprüche

1. Verfahren zur Abtrennung von in Isobuttersäure oder ihren Estern in Mengen von etwa 5 bis 10 ppm bis etwa 1 Gew.-% enthaltenem Fluorwasserstoff durch Behandeln des Gemisches mit einer Base,
dadurch gekennzeichnet,
daß die Abtrennung mit einem organischen Anionenaustauscherharz als Base durchgeführt wird.

2. Verfahren nach Anspruch 1, dadurch gekennzeichnet, daß die Abtrennung mit einem schwach basischen organischen Anionenaustauscherharz durchgeführt wird.

3. Verfahren nach den Ansprüchen 1 und 2, dadurch gekennzeichnet, daß die Abtrennung mit einem Anionenaustauscherharz auf Polystyrolbasis durchgeführt wird.

4. Verfahren nach den Ansprüchen 1 bis 3, dadurch gekennzeichnet, daß die Abtrennung mit einem Anionenaustauscherharz, das eine tertiäre Aminogruppe als funktionelle Gruppe enthält durchgeführt wird.

5. Verfahren nach den Ansprüchen 1 bis 4, dadurch gekennzeichnet, daß die Trennung am Harz bei Temperaturen von 0 Grad C und 90 Grad C, insbesondere zwischen 5 Grad C und 60 Grad C, vor allem zwischen 10 Grad C und 40 Grad C durchgeführt wird.

## Claims

1. Process for separating hydrogen fluoride contained in isobutyric acid or in the esters thereof in amounts of from about 5 to 10 ppm up to about 1 wt.-%, by treating the mixture with a base,
characterised in that
the separation is carried out using an organic anion exchanger resin as base.

2. A process according to claim 1, characterised in that the separation is carried out with a weakly basic organic anion exchanger resin.

3. A process according to claims 1 and 2, characterised in that the separation is carried out with an anion exchange resin based on polystyrene.

4. A process according to claims 1 to 3, characterised in that the separation is carried out with an anion exchange resin which contains a tertiary amino group as the functional group.

5. A process according to claims 1 to 4, characterised in that the separation is carried out on the resin at temperatures of from 0°C to 90°C, more particularly between 5°C and 60°C and most especially between 10°C and 40°C.

## Revendications

1. Procédé de séparation de l'acide fluorhydrique contenu dans des proportions d'environ 5-10 ppm à environ 1% en poids dans de l'acide isobutyrique ou ses esters, par traitement du mélange par une base,
caractérisé en ce que
la séparation est effectuée avec, en tant que base, une résine échangeuse d'anions organique.

2. Procédé selon la revendication 1, caractérisé en ce que la réparation est effectuée avec une résine échangeuse d'anions organique, faiblement basique.

3. Procédé selon la revendication 1 ou 2, caractérisé en ce que la séparation est effectuée avec une résine échangeuse d'anions à base de polystyrène.

4. Procédé selon l'une quelconque des revendications 1 à 3, caractérisé en ce que la séparation est effectuée avec une résine échangeuse d'anions qui contient un groupement amino tertiaire en tant que groupement fonctionnel.

5. Procédé selon l'une quelconque des revendications 1 à 4, caractérisé en ce que la séparation est effectuée sur la résine à des températures comprises entre 0°C et 90°C, en particulier entre 5°C et 60°C, et notamment entre 10°C et 40°C.